# EUROPEAN PATENT SPECIFICATION

(11) **EP 4 070 111 B1**
(45) Date of publication and mention of the grant of the patent: **21.08.2024**
(21) Application number: 20819704.6
(22) Date of filing: 02.12.2020
(51) Int. Cl.: G01N 33/574, G01N 33/68

(54) **USE OF CELL FREE NUCLEOSOMES AS BIOMARKERS**
VERWENDUNG VON ZELLFREIEN NUKLEOSOMEN ALS BIOMARKER
UTILISATION DE NUCLÉOSOMES ACELLULAIRES EN TANT QUE BIOMARQUEURS

(30) Priority: 02.12.2019 US 201962942596 P; 06.10.2020 US 202063088408 P
(43) Date of publication of application: 12.10.2022
(73) Proprietor: Belgian Volition Srl, 5032 Isnes (BE)
(72) Inventor: MICALLEF, Jacob Vincent, 5032 Isnes (BE); WILSON-ROBLES, Heather, 5032 Isnes (BE); ECCLESTON, Mark Edward, 5032 Isnes (BE); HERZOG, Marielle Chantal Andree, 5032 Isnes (BE); TERRELL, Jason Bradley, 5032 Isnes (BE)
(74) Representative: Sagittarius IP
(86) International application number: PCT/EP2020/084328
(87) International publication number: WO 2021/110776

(56) References cited:
- EP-A1- 2 282 208
- EP-B1- 2 282 208
- WO-A1-2016/067029
- WO-A1-2017/001863
- WO-A1-2017/001864
- WO-A1-2017/068359
- WO-A1-2019/169263
- WO-A2-2005/124337
- WO-A2-2013/030578
- WO-A2-2013/084002
- US-A1- 2013 230 858
- US-A1- 2014 107 039
- HOLDENRIEDER STEFAN: "Histone modifications on circulating nucleosomes as a new class of cancer biomarkers", TUMOR BIOLOGY, KARGER, BASEL, CH, vol. 33, no. Suppl. 1, 1 October 2012 (2012-10-01), pages S26 - S27, XP009167379, ISSN: 1010-4283
- BOHENG LI ET AL: "Abstract", JOURNAL OF HEMATOLOGY & ONCOLOGY, vol. 12, no. 1, 21 November 2019 (2019-11-21), XP055769325, DOI: 10.1186/s13045-019-0814-6
- M. T. MCCABE ET AL: "Mutation of A677 in histone methyltransferase EZH2 in human B-cell lymphoma promotes hypertrimethylation of histone H3 on lysine 27 (H3K27)", PROCEEDINGS OF THE NATIONAL ACADEMY OF SCIENCES, vol. 109, no. 8, 21 February 2012 (2012-02-21), pages 2989 - 2994, XP055075955, ISSN: 0027-8424, DOI: 10.1073/pnas.1116418109
- BOHENG LI ET AL: "EZH2 abnormalities in lymphoidmalignancies: underlying mechanisms andtherapeutic implications", JOURNAL OF HEMATOLOGY & ONCOLOGY, vol. 12, no. 1, 21 November 2019 (2019-11-21), XP055769518, DOI: 10.1186/s13045-019-0814-6
- STEFAN HOLDENRIEDER ET AL: "Nucleosomes in serum of patients with benign and malignant diseases", INTERNATIONAL JOURNAL OF CANCER, vol. 95, no. 2, 20 March 2001 (2001-03-20), US, pages 114 - 120, XP055566675, ISSN: 0020-7136, DOI: 10.1002/1097-0215(20010320)95:2<114::AID-IJC1020>3.0.CO;2-Q
- STEFAN HOLDENRIEDER ET AL: "Clinical Relevance of Circulating Nucleosomes in Cancer", ANNALS OF THE NEW YORK ACADEMY OF SCIENCES, vol. 1137, no. 1, 1 August 2008 (2008-08-01), pages 180 - 189, XP055046551, ISSN: 0077-8923, DOI: 10.1196/annals.1448.012
- HEIDI SCHWARZENBACH ET AL: "Cell-free nucleic acids as biomarkers in cancer patients", NATURE REVIEWS CANCER, vol. 11, no. 6, 12 May 2011 (2011-05-12), London, pages 426 - 437, XP055247315, ISSN: 1474-175X, DOI: 10.1038/nrc3066
- HERRANZ MICHEL ET AL: "DNA methylation and histone modifications in patients with cancer: potential prognostic and therapeutic targets", ANTIBODY-DRUG CONJUGATES; IN: METHODS IN MOLECULAR BIOLOGY; ISSN 1064-3745; VOL. 263; [METHODS IN MOLECULAR BIOLOGY; ISSN 1064-3745], HUMANA PRESS, US, vol. 361, 1 January 2007 (2007-01-01), pages 25 - 62, XP009115201, ISBN: 978-1-62703-541-5

## Description

### FIELD OF THE INVENTION

The present invention relates to cell free nucleosomes containing histone isoform H3.1 as biomarkers in plasma samples for vascular or haematological cancers.

### BACKGROUND OF THE INVENTION

Haematological cancers are the types of cancer affecting blood, bone marrow and lymph nodes. They are referred to as leukaemia, lymphoma and myeloma depending on the type of cell affected. Leukaemia is cancer of the blood cells which usually starts in the bone marrow and travels through the bloodstream. In leukaemia, the bone marrow produces mutated cells and spreads them into the blood, where they grow and crowd out healthy blood cells. Lymphoma diseases affect the cells in the lymphatic system. In lymphomas, immune cells called lymphocytes grow out of control and collect in lymph nodes, the spleen, in other lymph tissues or in neighbouring organs. Myeloma, also known as multiple myeloma, develops in the bone marrow and affects plasma cells, which produce antibodies that attack infections and diseases. Examples of blood cancers include Acute Lymphoblastic Leukaemia (ALL), Acute Myeloid Leukaemia (AML), Hodgkin Lymphoma (HL) and Non-Hodgkin Lymphoma (NHL).

References to "acute leukaemia" means the cancer progresses quickly and aggressively, usually requiring immediate treatment. ALL involves the development of large numbers of immature lymphocytes which are unable to fight infection. This causes the patient to have less room for healthy white blood cells, red blood cells, and platelets in the circulation. As a result, the patient usually suffers from a weakened immune system and the symptoms of anaemia, such as tiredness, breathlessness and an increased risk of excessive bleeding. The risk for developing ALL is highest in children younger than 5 years of age and it is the most common type of leukaemia that affects children. The risk then declines slowly until the mid-20s, and begins to rise again slowly after age 50. Overall, about 4 of every 10 cases of ALL are in adults.

AML affects myeloblasts which results in the accumulation of abnormal monocytes and granulocytes in the bone marrow. AML may also affect myeloid stem cells resulting in abnormal red blood cells or platelets. As with ALL, this causes the patient to have lower levels of healthy white blood cells, red blood cells, and platelets in the circulation. AML is one of the most common types of leukaemia in adults and the average age at diagnosis is 68.

HL and NHL are the two main types of lymphoma. HL has a particular appearance under the microscope and contains cells called Reed-Sternberg cells (a type of B lymphocyte that has become cancerous), whereas NHL looks different under the microscope and does not contain Reed-Sternberg cells. Most lymphomas are NHL and only about 1 in 5 are HL. NHL is a cancer affecting lymphocytes and usually starts in lymph nodes or lymph tissue. It is one of the more common cancers among children, teens and young adults.

Current methods of diagnosing leukaemia and myeloma involve obtaining a complete blood count (CBC) test to identify abnormal levels of white blood cells relative to red blood cells and platelets. However, an elevated white blood cell count (WBC) is not specific to patients with a haematological malignancy; it can also be the result of an ongoing response to infection or other inflammatory process. For lymphoma, an X-ray, CT or PET scan can be used to detect swollen lymph nodes, however this is also non-specific.

In order to confirm a diagnosis of a haematological cancer, a bone marrow or lymph node biopsy is required. Therefore overdiagnosis of haematological cancers at an early stage in the diagnostic process can lead to unnecessary biopsies which are invasive, potentially hazardous and relatively costly to healthcare providers. Cytogenetics analysis and/or immunophenotyping can also be used to confirm a haematological cancer diagnosis, however these methods are expensive to perform and therefore are typically only used at a late stage of the diagnostic process.

Human angiosarcoma is a rare vascular cancer affecting the endothelial cells lining the blood vessels. Canine hemangiosarcoma is similarly a cancer involving proliferation of the vascular endothelium or the blood vessel walls and is a common canine cancer which is also difficult to diagnose. Canine cancers are more common than human cancers generally both due to the genetic effects of inbreeding and because dogs have a shorter life-span than humans and commonly contract cancer at 8 years of age or above. Detection and diagnosis of cancer in animal subjects presents additional difficulties over diagnosis of human cancers. Cancer detection in non-human animals often involves scanning, for example by Magnetic Resonance Imaging or MRI scanning, but animals will not remain still for the time required for scanning and must therefore be anaesthetised. Pet health insurance is rare and may not cover cancer diagnostics or therapy making these test financially out of reach for many pet owners. In addition, human blood tests for cancer marker proteins do not normally detect animal proteins so there are fewer available blood tests in veterinary oncology.

Holdenrieder et al. (2001) Int J Cancer 95: 114-120 previously described detecting the level of nucleosomes in serum samples of patients with benign and malignant diseases. However, the results presented for serum samples did not imply that there was any distinction between the level for haematological cancers, such as lymphoma, compared to the other cancer types tested. The epigenetic composition of circulating cell free nucleosomes in terms of their histone modification, histone variant, DNA modification and adduct content have also been investigated as blood based biomarkers in cancer, see WO 2005/019826, WO 2013/030577, WO 2013/030579 and WO 2013/084002.

WO2019/169263 describes the use of recombinant/semi-synthetic nucleosomes carrying histone and/or DNA modifications as a reference standard for quantification of covalently modified nucleosomes from a biological sample. WO2017/068359 describes methods for detecting and measuring the presence of cell free mono-nucleosomes and oligo-nucleosomes that contain histone H1 or a histone H1 modification, variant or isoform, and the use of such measurements for the detection and diagnosis of disease.

There remains a need in the art to provide simple, cost-effective diagnostic methods for vascular or haematological cancers, especially those able to distinguish from patients with other types of disease or non-vascular or non-haematological cancer, but who may present with similar symptoms.

### SUMMARY OF THE INVENTION

According to a first aspect, there is provided a use of a H3.1 histone isoform of a cell free nucleosome as a biomarker in a plasma sample, for the diagnosis or detecting of a vascular or haematological cancer.

According to a further aspect, there is provided a use of a H3.1 histone isoform of a cell free nucleosome as a biomarker in a plasma sample, for the diagnosis or detection of a haematological cancer.

According to a further aspect, there is provided a use of a H3.1 histone isoform of a cell free nucleosome as a biomarker in a plasma sample, for the diagnosis or detecting of a vascular cancer.

According to a further aspect, there is provided a method for diagnosing or detecting a vascular or haematological cancer which comprises the steps of:
(i) contacting a plasma sample obtained from the subject with a binding agent to detect or measure cell free nucleosomes containing histone isoform H3.1; and
(ii) using the level of cell free nucleosomes containing histone isoform H3.1 detected to diagnose the subject with the vascular or haematological cancer.

According to a further aspect, there is provided a method for determining the prognosis of a subject with a vascular or haematological cancer, which comprises the steps of:
(i) contacting a plasma sample obtained from the subject with a binding agent to detect or measure cell free nucleosomes containing histone isoform H3.1; and
(ii) using the level of cell free nucleosomes containing histone isoform H3.1 detected as indicative of the prognosis of said vascular or haematological cancer.

According to a further aspect, there is provided a method for monitoring the efficacy of a therapy in a subject having, suspected of having, or being predisposed to a vascular or haematological cancer, which comprises the steps of:
(i) contacting a plasma sample obtained from the subject with a binding agent to detect or measure cell free nucleosomes containing histone isoform H3.1; and
(ii) comparing the level of cell free nucleosomes containing histone isoform H3.1 detected with an earlier plasma sample taken from said subject to determine the efficacy of said therapy.

### BRIEF DESCRIPTION OF THE FIGURES

**FIGURE 1****:** Concentration of cell free nucleosomes containing H3.1 in plasma samples obtained from healthy human subjects and human subjects with Acute Lymphocytic Leukaemia (ALL), Acute Myeloid Leukaemia (AML) and Non-Hodgkin Lymphoma (NHLym).
**FIGURE 2****:** Concentration of cell free nucleosomes containing H3.1 in plasma samples obtained from human subjects with different types of cancer.
**FIGURE 3****:** ELISA optical density (OD) results of cell free nucleosomes containing H3 citrullination (H3cit) in plasma samples from healthy human subjects in comparison to (A) results from all types of haematological cancer tested, and (B) results from human subjects with ALL, AML and NHL.
**FIGURE 4****:** ELISA OD results of cell free nucleosomes containing H3K27Me3 in plasma samples from healthy human subjects in comparison to (A) results from all types of haematological cancer tested, and (B) results from human subjects with ALL, AML and NHL.
**FIGURE 5****:** ELISA relative light unit (RLU) results of cell free nucleosomes containing H4 pan acetylation (H4panAc) in plasma samples from healthy human subjects in comparison to (A) results from all types of haematological cancer tested, and (B) results from human subjects with ALL, AML and NHL.
**FIGURE 6****:** (A) ELISA results of cell free nucleosomes containing histone isoform H3.1 (ng/ml) in plasma samples taken from healthy canine subjects in comparison to canine subjects diagnosed with lymphoma, and (B) ROC curve showing detection of canine lymphoma using H3.1 results.
**FIGURE 7****:** (A) ELISA results of cell free nucleosomes containing histone isoform H3.1 (ng/ml) in plasma samples taken from healthy canine subjects in comparison to canine subjects diagnosed with hemangiosarcoma, and (B) ROC curve showing detection of canine hemangiosarcoma using H3.1 results.
**FIGURE 8****:** ELISA results of cell free nucleosomes containing histone isoform H3.1 (ng/ml) and CRP (µg/ml) in plasma samples taken serially from 2 canine subjects under treatment for hemangiosarcoma.
**FIGURE 9****:** ELISA results of cell free nucleosomes containing histone isoform H3.1 (ng/ml) and CRP (µg/ml) in plasma samples taken serially from 2 canine subjects under treatment for lymphoma.

### DETAILED DESCRIPTION

According to a first aspect, there is provided a use of a H3.1 histone isoform of a cell free nucleosome as a biomarker in a plasma sample, for the diagnosis or detection of a vascular or haematological cancer. In particular, the cancer is a haematological cancer.

The nucleosome is the basic unit of chromatin structure and consists of a protein complex of eight highly conserved core histones (comprising of a pair of each of the histones H2A, H2B, H3, and H4). Around this complex is wrapped approximately 146 base pairs of DNA. Another histone, H1 or H5, acts as a linker and is involved in chromatin compaction. The DNA is wound around consecutive nucleosomes in a structure often said to resemble "beads on a string" and this forms the basic structure of open or euchromatin. In compacted or heterochromatin this string is coiled and super coiled into a closed and complex structure (Herranz and Esteller (2007) Methods Mol. Biol. 361: 25-62).

References to "nucleosome" may refer to "cell free nucleosome" when detected in body fluid samples. It will be appreciated that the term cell free nucleosome throughout this document is intended to include any cell free chromatin fragment that includes one or more nucleosomes. "Epigenetic features", "epigenetic signal features" or "epigenetic signal structures" of a cell free nucleosome as referred herein may comprise, without limitation, one or more histone post-translational modifications, histone isoforms, modified nucleotides and/or proteins bound to a nucleosome in a nucleosome-protein adduct.

It will be understood that the cell free nucleosome may be detected by binding to a component thereof. The term "component thereof' as used herein refers to a part of the nucleosome, *i.e.* the whole nucleosome does not need to be detected. The component of the cell free nucleosomes may be selected from the group consisting of: a histone protein (*i.e.* histone H1, H2A, H2B, H3 or H4), a histone post-translational modification, a histone variant or isoform, a protein bound to the nucleosome (*i.e.* a nucleosome-protein adduct), a DNA fragment associated with the nucleosome and/or a modified nucleotide associated with the nucleosome. According to the present invention, the component thereof is histone (isoform) H3.1.

Methods and uses of the invention may measure the level of (cell free) nucleosomes *per se.* References to "nucleosomes *per se*" refers to the total nucleosome level or concentration present in the sample, regardless of any epigenetic features the nucleosomes may or may not include. Detection of the total nucleosome level typically involves detecting a histone protein common to all nucleosomes, such as histone H4. Therefore, nucleosomes *per se* may be measured by detecting a core histone protein, such as histone H4. As described herein, histone proteins form structural units known as nucleosomes which are used to package DNA in eukaryotic cells. As previously reported in WO 2016/067029, particular histone variants, such as histone H3.1, H3.2 or H3t, may be used to isolate cell free nucleosomes originating from tumour cells. Therefore, the total level of cell free nucleosomes of tumour origin may be detected.

Normal cell turnover in adult humans involves the creation by cell division of some 10¹¹ cells daily and the death of a similar number, mainly by apoptosis. During the process of apoptosis chromatin is broken down into mononucleosomes and oligonucleosomes which are released from the cells. Under normal conditions the levels of circulating nucleosomes found in healthy subjects is reported to be low. Elevated levels are found in subjects with a variety of conditions including many cancers, auto-immune diseases, inflammatory conditions, stroke and myocardial infarction (Holdenreider & Stieber (2009) Crit Rev Clin Lab Sci, 46(1): 1-24).

Current nucleosome ELISA methods are used primarily in cell culture, usually as a method to detect apoptosis (Salgame et al. (1997) Nucleic Acids Res, 25(3): 680-681; Holdenrieder *et al.* (2001) *supra*; van Nieuwenhuijze et al. (2003) Ann Rheum Dis, 62: 10-14), but are also used for the measurement of circulating cell free nucleosomes in serum and plasma (Holdenrieder *et al.* (2001)). Cell free serum and plasma nucleosome levels released into the circulation by dying cells have been measured by ELISA methods in studies of a number of different cancers to evaluate their use as a potential biomarker. Mean circulating nucleosome levels are reported to be high in most, but not all, cancers studied. However, patients with malignant tumours are reported to have serum nucleosome concentrations that varied considerably and some patients with advanced tumour disease were found to have low circulating nucleosome levels, within the range measured for healthy subjects (Holdenrieder *et al.* (2001)).

The cell free nucleosome may be a mononucleosome or oligonucleosome, or a mixture thereof.

Mononucleosomes and oligonucleosomes can be detected by Enzyme-Linked ImmunoSorbant Assay (ELISA) and several methods have been reported (*e.g*. Salgame *et al.* (1997); Holdenrieder *et al.* (2001); van Nieuwenhuijze *et al.* (2003)). These assays typically employ an anti-histone antibody (for example anti-H2B, anti-H3 or anti-H1, H2A, H2B, H3 and H4) as capture antibody and an anti-DNA or anti-H2A-H2B-DNA complex antibody as detection antibody.

Circulating nucleosomes are not a homogeneous group of protein-nucleic acid complexes. Rather, they are a heterogeneous group of chromatin fragments originating from the digestion of chromatin on cell death and include an immense variety of epigenetic structures including particular histone isoforms (or variants), post-translational histone modifications, nucleotides or modified nucleotides, and protein adducts. It will be clear to those skilled in the art that an elevation in nucleosome levels will be associated with elevations in some circulating nucleosome subsets containing particular epigenetic signals including nucleosomes comprising particular histone isoforms (or variants), comprising particular post-translational histone modifications, comprising particular nucleotides or modified nucleotides and comprising particular protein adducts. Assays for these types of chromatin fragments are known in the art (for example, see WO 2005/019826, WO 2013/030579, WO 2013/030578, WO 2013/084002).

It will be understood that the terms "epigenetic signal structure" and "epigenetic feature" are used interchangeably herein. They refer to particular features of the nucleosome that may be detected. In one embodiment, the epigenetic feature of the nucleosome is selected from the group consisting of: a post-translational histone modification, a histone variant, a particular nucleotide and a protein adduct.

The term "histone variant" and "histone isoform" may be used interchangeably herein. The structure of the nucleosome can also vary by the inclusion of alternative histone isoforms or variants which are different gene or splice products and have different amino acid sequences. Many histone isoforms are known in the art. Histone variants can be classed into a number of families which are subdivided into individual types. The nucleotide sequences of a large number of histone variants are known and publicly available for example in the National Human Genome Research Institute NHGRI Histone Database (Mariño-Ramírez et al. The Histone Database: an integrated resource for histones and histone fold-containing proteins. Database Vol.2011. and http://genome.nhgri.nih.gov/histones/complete.shtml), the GenBank (NIH genetic sequence) Database, the EMBL Nucleotide Sequence Database and the DNA Data Bank of Japan (DDBJ). For example, variants of histone H2 include H2A1, H2A2, mH2A1, mH2A2, H2AX and H2AZ. In another example, histone isoforms of H3 include H3.1, H3.2 and H3t.

According to the present invention, the histone isoform is H3.1. As shown in the examples presented herein, H3.1 was effective at distinguishing subjects with vascular or haematological cancer from healthy subjects. H3.1 was particularly effective at identifying patients with lymphoma because 84% of patients with NHL were distinguished from healthy subjects at 90% specificity (see Table 1).

The structure of nucleosomes can vary by post translational modification (PTM) of histone proteins. PTM of histone proteins typically occurs on the tails of the core histones and common modifications include acetylation, methylation or ubiquitination of lysine residues as well as methylation of arginine residues and phosphorylation of serine residues and many others. Many histone modifications are known in the art and the number is increasing as new modifications are identified (Zhao and Garcia, 2015 Cold Spring Harb Perspect Biol, 7: a025064). Histone PTMs include a histone PTM of a core nucleosome, *e.g*. H3, H2A, H2B or H4, in particular H3, H2A or H2B. Examples of such PTMs are described in WO 2005/019826.

For example, the post translational modification may include acetylation, methylation, which may be mono-, di-or tri-methylation, phosphorylation, ribosylation, citrullination, ubiquitination, hydroxylation, glycosylation, nitrosylation, glutamination and/or isomerisation (see Ausio (2001) Biochem Cell Bio 79: 693). As shown in the examples presented herein, H3citwas the most effective histone PTM at distinguishing subjects with vascular or haematological cancer from healthy subjects.

A group or class of related histone post translational modifications (rather than a single modification) may also be detected. A typical example, without limitation, would involve a 2-site immunoassay employing one antibody or other selective binder directed to bind to nucleosomes and one antibody or other selective binder directed to bind the group of histone modifications in question. Examples of such antibodies directed to bind to a group of histone modifications would include, for illustrative purposes without limitation, anti-pan-acetylation antibodies (*e.g*. a Pan-acetyl H4 antibody [H4panAc]), anti-citrullination antibodies or anti-ubiquitin antibodies.

In addition to the epigenetic signalling mediated by nucleosome histone isoform and PTM composition, nucleosomes also differ in their nucleotide and modified nucleotide composition. Global DNA hypomethylation is a hallmark of cancer cells and some nucleosomes may comprise more 5-methylcytosine residues (or 5-hydroxymethylcytosine residues or other nucleotides or modified nucleotides) than other nucleosomes.

A further type of circulating nucleosome subset is nucleosome protein adducts. It has been known for many years that chromatin comprises a large number of non-histone proteins bound to its constituent DNA and/or histones. These chromatin associated proteins are of a wide variety of types and have a variety of functions including transcription factors, transcription enhancement factors, transcription repression factors, histone modifying enzymes, DNA damage repair proteins and many more. These chromatin fragments including nucleosomes and other non-histone chromatin proteins or DNA and other non-histone chromatin proteins are described in the art.

A protein may be adducted to the nucleosome, for example: a transcription factor, a High Mobility Group Protein or chromatin modifying enzyme. References to "transcription factor" refer to proteins that bind to DNA and regulate gene expression by promoting (*i.e*. activators) or suppressing (*i.e*. repressors) transcription. Transcription factors contain one or more DNA-binding domains (DBDs), which attach to specific sequences of DNA adjacent to the genes that they regulate. All of the circulating nucleosomes and nucleosome moieties, types or subgroups described herein may be useful in the present invention.

It will be understood that more than one epigenetic feature of cell free nucleosomes may be detected in methods and uses of the invention. Multiple biomarkers may be used as a combined biomarker. Therefore, in one embodiment, the use comprises more than one epigenetic feature of cell free nucleosomes as a combined biomarker. The epigenetic features may be the same type (*e.g*. PTMs, histone isoforms, nucleotides or protein adducts) or different types (*e.g*. a PTM in combination with a histone isoform). For example, a post-translational histone modification and a histone variant may be detected (*i.e*. more than one type of epigenetic feature is detected). Alternatively, or additionally, more than one type of post-translational histone modification is detected, or more than one type of histone isoform is detected. In one embodiment, the combined biomarker is H3.1 and H3cit. In an alternative embodiment, the combined biomarker is H3.1 and H3K27Me3.

The term "biomarker" means a distinctive biological or biologically derived indicator of a process, event, or condition. Biomarkers can be used in methods of diagnosis, *e.g*. clinical screening, and prognosis assessment and in monitoring the results of therapy, identifying patients most likely to respond to a particular therapeutic treatment, drug screening and development. Biomarkers and uses thereof are valuable for identification of new drug treatments and for discovery of new targets for drug treatment.

Methods and uses described herein may be tested in body fluid samples, in particular blood, serum or plasma samples. Preferably, plasma samples are used. Plasma samples may be collected in collection tubes containing one or more anticoagulants such as ethylenediamine tetraacetic acid (EDTA), heparin, or sodium citrate, in particular EDTA.

### Haematological cancers

Haematological cancers are cancers of the blood, therefore may also be referred to as "blood cancers". There are 3 principal types of haematological cancers: leukaemias, which are caused by the rapid production of abnormal white blood cells; lymphomas which are caused by abnormal lymphoma cells; and myelomas, which is a cancer of the plasma cells.

In one embodiment the haematological cancer is selected from lymphoma, leukaemia, myeloma, chronic myeloproliferative disease, monoclonal gammopathy of uncertain significance, myelodysplastic syndrome and amyloidosis. In a further embodiment, the haematological cancer is selected from leukaemia or lymphoma.

Leukaemia affects white blood cells and can be classified by the type of white cell affected (myeloid or lymphatic) and by the way the disease progresses (acute or chronic). Several types of leukaemia have been identified including, but not limited to: Acute Lymphoblastic Leukaemia (ALL; which may also be referred to as Acute Lymphocytic Leukaemia), Acute Myeloid Leukaemia (AML), Acute Megakaryoblastic Leukaemia (AMKL), Acute Promyelocytic Leukaemia (APL), Childhood Acute Myeloid Leukaemia (C-AML), Childhood Acute Lymphocytic Leukaemia (C-ALL), Chronic Eosinophilic Leukaemia (CEL), Chronic Lymphocytic Leukaemia (CLL), Chronic Myeloid Leukaemia (CML), Chronic Myelomonocytic Leukaemia (CMML), Chronic Neutrophilic Leukaemia, Hairy Cell Leukaemia, Juvenile Myelomonocytic Leukaemia (JMML), Large Granular Lymphocytic Leukaemia (LGLL), T-cell Acute Lymphoblastic Leukaemia and Prolymphocytic Leukaemia.

In one embodiment, the leukaemia is an acute leukaemia, such as Acute Lymphocytic Leukaemia (ALL), Acute Myeloid Leukaemia (AML), Acute Megakaryoblastic Leukaemia (AMKL) or Acute Promyelocytic Leukaemia (APL). In a further embodiment, the leukaemia is selected from Acute Lymphocytic Leukaemia (ALL) and Acute Myeloid Leukaemia (AML). Alternatively, in one embodiment, the leukaemia is a chronic leukaemia, such as Chronic Eosinophilic Leukaemia (CEL), Chronic Lymphocytic Leukaemia (CLL), Chronic Myeloid Leukaemia (CML), Chronic Myelomonocytic Leukaemia (CMML) or Chronic Neutrophilic Leukaemia.

Both Hodgkin Lymphoma (HL) and Non-Hodgkin Lymphoma (NHL) are lymphomas. The majority of NHL patients are over the age of 55 when first diagnosed, whereas the median age for diagnosis of Hodgkin lymphoma is 39. In one embodiment, the lymphoma is Non-Hodgkin Lymphoma (NHL). NHL may arise in lymph nodes anywhere in the body, whereas HL typically begins in the upper body, such as the neck, chest or armpits.

Hodgkin lymphoma is often diagnosed at an early stage and is therefore considered one of the most treatable cancers. Non-Hodgkin lymphoma is typically not diagnosed until it has reached a more advanced stage, therefore methods of the invention find particular use in the diagnosis of NHL where there is a need to detect patients at an early stage of disease to improve treatment outcome.

### Other blood cancers

Angiosarcoma and Hemangiosarcoma are soft tissue cancers involving proliferation of the cells lining the vascular system, therefore may be referred to as "vascular cancers". Like haematopoietic cancers, these intimately associated with the vasculature and the affected cells are in direct contact with the circulating fluid of the blood. We have shown that these blood cancers are associated with very high levels of circulating nucleosomes which are similar to the levels observed in lymphoma.

### Detection and diagnosis methods

The invention provides methods which can be used in the detection or diagnosis of patients with vascular or haematological cancers. Therefore, according to a further aspect, there is provided a method for diagnosing or detecting a vascular or haematological cancer which comprises the steps of:
(i) contacting a plasma sample obtained from the subject with a binding agent to detect or measure cell free nucleosomes containing histone isoform H3.1; and
(ii) using the level of cell free nucleosomes containing histone isoform H3.1 detected to diagnose the subject with the vascular or haematological cancer.

According to a further aspect, there is provided a method for diagnosing or detecting a haematological cancer which comprises the steps of:
(i) contacting a plasma sample obtained from the subject with a binding agent to detect or measure cell free nucleosomes containing histone isoform H3.1; and
(ii) using the level of cell free nucleosomes containing histone isoform H3.1 detected to diagnose the subject with the haematological cancer.

Alternatively, according to a further aspect, there is provided a method for diagnosing or detecting a vascular cancer which comprises the steps of:
(i) contacting a plasma sample obtained from the subject with a binding agent to detect or measure cell free nucleosomes containing histone isoform H3.1; and
(ii) using the level of cell free nucleosomes containing histone isoform H3.1 detected to diagnose the subject with the vascular cancer.

According to a further aspect, there is provided a method for determining the prognosis of a subject with a vascular or haematological cancer, which comprises the steps of:
(i) contacting a plasma sample obtained from the subject with a binding agent to detect or measure cell free nucleosomes containing histone isoform H3.1; and
(ii) using the level of cell free nucleosomes containing histone isoform H3.1 detected as indicative of the prognosis of said vascular or haematological cancer.

If a subject is determined to not have a vascular or haematological cancer, then the invention may still be used for the purposes of monitoring disease progression. For example, if the use comprises a sample from a subject determined not to have a vascular or haematological cancer, then the biomarker level measurements can be repeated at another time point to establish if the biomarker level has changed.

According to a further aspect, there is provided a method for monitoring the efficacy of a therapy in a subject having, suspected of having, or being predisposed to a vascular or haematological cancer, which comprises the steps of:
(i) contacting a plasma sample obtained from the subject with a binding agent to detect or measure cell free nucleosomes containing histone isoform H3.1; and
(ii) comparing the level of cell free nucleosomes containing histone isoform H3.1 detected with an earlier plasma sample taken from said subject to determine the efficacy of said therapy.

Detecting and/or quantifying may be performed directly on the purified or enriched nucleosome sample, or indirectly on an extract therefrom, or on a dilution thereof. Quantifying the amount of the biomarker present in a sample may include determining the concentration of the biomarker present in the sample. Uses and methods of detecting, monitoring and of diagnosis according to the invention described herein are useful to confirm the existence of a disease, to monitor development of the disease by assessing onset and progression, or to assess amelioration or regression of the disease. Uses and methods of detecting, monitoring and of diagnosis are also useful in methods for assessment of clinical screening, prognosis, choice of therapy, evaluation of therapeutic benefit, *i.e.* for drug screening and drug development.

The detection or measurement may comprise an immunoassay, immunochemical, mass spectroscopy, chromatographic, chromatin immunoprecipitation or biosensor method. In particular, detection and/or measurement may comprise a 2-site immunoassay method for nucleosome moieties. Such a method is preferred for the measurement of nucleosomes or nucleosome incorporated epigenetic features *in situ* employing two anti-nucleosome binding agents or an anti-nucleosome binding agent in combination with an anti-histone modification or anti-histone variant or anti-DNA modification or anti-adducted protein detection binding agent. Also, detection and/or measurement may comprise a 2-site immunoassay employing a labelled anti-nucleosome detection binding agent in combination with an immobilized anti-histone modification or anti-histone variant or anti-DNA modification or anti-adducted protein binding agent.

Detecting or measuring the level of the biomarker(s) may be performed using one or more reagents, such as a suitable binding agent. For example, the one or more binding agents may comprise a ligand or binder specific for the desired biomarker, *e.g*. nucleosomes or component part thereof, an epigenetic feature of a nucleosome, a structural/shape mimic of the nucleosome or component part thereof, optionally in combination with one or more interleukins.

It will be clear to those skilled in the art that the terms "antibody", "binder" or "ligand" as used herein are not limiting but are intended to include any binder capable of binding to particular molecules or entities and that any suitable binder can be used in the method of the invention. It will also be clear that the term "nucleosomes" is intended to include mononucleosomes and oligonucleosomes and any protein-DNA chromatin fragments that can be analysed in fluid media.

Methods of detecting biomarkers are known in the art. The reagents may comprise one or more ligands or binders, for example, naturally occurring or chemically synthesised compounds, capable of specific binding to the desired target. A ligand or binder may comprise a peptide, an antibody or a fragment thereof, or a synthetic ligand such as a plastic antibody, or an aptamer or oligonucleotide, capable of specific binding to the desired target. The antibody can be a monoclonal antibody or a fragment thereof. It will be understood that if an antibody fragment is used then it retains the ability to bind the biomarker so that the biomarker may be detected (in accordance with the present invention). A ligand/binder may be labelled with a detectable marker, such as a luminescent, fluorescent, enzyme or radioactive marker; alternatively or additionally a ligand according to the invention may be labelled with an affinity tag, *e.g.* a biotin, avidin, streptavidin or His (*e.g*. hexa-His) tag. Alternatively, ligand binding may be determined using a label-free technology for example that of ForteBio Inc.

The term "detecting" or "diagnosing" as used herein encompasses identification, confirmation, and/or characterisation of a disease state. Methods of detecting, monitoring and of diagnosis according to the invention are useful to confirm the existence of a disease, to monitor development of the disease by assessing onset and progression, or to assess amelioration or regression of the disease. Methods of detecting, monitoring and of diagnosis are also useful in methods for assessment of clinical screening, prognosis, choice of therapy, evaluation of therapeutic benefit, *i.e.* for drug screening and drug development.

In one embodiment, the method described herein is repeated on multiple occasions. This embodiment provides the advantage of allowing the detection results to be monitored over a time period. Such an arrangement will provide the benefit of monitoring or assessing the efficacy of treatment of a disease state. Such monitoring methods of the invention can be used to monitor onset, progression, stabilisation, amelioration, relapse and/or remission.

In monitoring methods, test samples may be taken on two or more occasions. The method may further comprise comparing the level of the biomarker(s) present in the test sample with one or more control(s) and/or with one or more previous test sample(s) taken earlier from the same test subject, *e.g*. prior to commencement of therapy, and/or from the same test subject at an earlier stage of therapy. The method may comprise detecting a change in the nature or amount of the biomarker(s) in test samples taken on different occasions.

A change in the level of the biomarker in the test sample relative to the level in a previous test sample taken earlier from the same test subject may be indicative of a beneficial effect, *e.g*. stabilisation or improvement, of said therapy on the disorder or suspected disorder. Furthermore, once treatment has been completed, the method of the invention may be periodically repeated in order to monitor for the recurrence of a disease.

Methods for monitoring efficacy of a therapy can be used to monitor the therapeutic effectiveness of existing therapies and new therapies in human subjects and in non-human animals (*e.g*. in animal models). These monitoring methods can be incorporated into screens for new drug substances and combinations of substances.

In a further embodiment the monitoring of more rapid changes due to fast acting therapies may be conducted at shorter intervals of hours or days.

Diagnostic or monitoring kits (or panels) are provided for performing methods of the invention. Such kits will suitably comprise one or more ligands for detection and/or quantification of the biomarker, and/or a biosensor, and/or an array as described herein, optionally together with instructions for use of the kit.

Also described is a kit for detecting the presence of a disease state, comprising a biosensor capable of detecting and/or quantifying one or more of the biomarkers as defined herein. As used herein, the term "biosensor" means anything capable of detecting the presence of the biomarker. Examples of biosensors are described herein. Biosensors may comprise a ligand binder or ligands, as described herein, capable of specific binding to the biomarker. Such biosensors are useful in detecting and/or quantifying a biomarker.

Suitably, biosensors for detection of one or more biomarkers combine biomolecular recognition with appropriate means to convert detection of the presence, or quantitation, of the biomarker in the sample into a signal. Biosensors can be adapted for "alternate site" diagnostic testing, *e.g*. in the ward, outsubjects' department, surgery, home, field and workplace. Biosensors to detect one or more biomarkers include acoustic, plasmon resonance, holographic, Bio-Layer Interferometry (BLI) and microengineered sensors. Imprinted recognition elements, thin film transistor technology, magnetic acoustic resonator devices and other novel acousto-electrical systems may be employed in biosensors for detection of the one or more biomarkers.

Biomarkers for detecting the presence of a disease are essential targets for discovery of novel targets and drug molecules that retard or halt progression of the disorder. As the level of the biomarker is indicative of disorder and of drug response, the biomarker is useful for identification of novel therapeutic compounds in *in vitro* and/or *in vivo* assays. Biomarkers described herein can be employed in methods for screening for compounds that modulate the activity of the biomarker.

Thus, also described, is the use of a binder or ligand, as described, which can be a peptide, antibody or fragment thereof or aptamer or oligonucleotide directed to a biomarker; or the use of a biosensor, or an array, or a kit, to identify a substance capable of promoting and/or of suppressing the generation of the biomarker.

The immunoassays described herein include any method employing one or more antibodies or other specific binders directed to bind to the biomarkers defined herein. Immunoassays include 2-site immunoassays or immunometric assays employing enzyme detection methods (for example ELISA), fluorescence labelled immunometric assays, time-resolved fluorescence labelled immunometric assays, chemiluminescent immunometric assays, immunoturbidimetric assays, particulate labelled immunometric assays and immunoradiometric assays as well as single-site immunoassays, reagent limited immunoassays, competitive immunoassay methods including labelled antigen and labelled antibody single antibody immunoassay methods with a variety of label types including radioactive, enzyme, fluorescent, time-resolved fluorescent and particulate labels. All of said immunoassay methods are well known in the art, see for example Salgame *et al.* (1997) and van Nieuwenhuijze *et al.* (2003).

Identifying, detecting and/or quantifying can be performed by any method suitable to identify the presence and/or amount of a specific protein in a biological sample from a subject or a purification or extract of a biological sample or a dilution thereof. In particular, quantifying may be performed by measuring the concentration of the target in the sample or samples. Biological samples that may be tested in a method of the invention include those as defined hereinbefore. The samples can be prepared, for example where appropriate diluted or concentrated, and stored in the usual manner. The present invention finds particular use in plasma samples which may be obtained from the subject.

Identification, detection and/or quantification of biomarkers may be performed by detection of the biomarker or of a fragment thereof, *e.g*. a fragment with C-terminal truncation, or with N-terminal truncation. Fragments are suitably greater than 4 amino acids in length, for example 5, 6, 7, 8, 9, 10, 11, 12, 13, 14, 15, 16, 17, 18, 19, or 20 amino acids in length. It is noted in particular that peptides of the same or related sequence to that of histone tails are particularly useful fragments of histone proteins.

For example, detecting and/or quantifying can be performed by one or more method(s) selected from the group consisting of: SELDI (-TOF), MALDI (-TOF), a 1-D gel-based analysis, a 2-D gel-based analysis, Mass spec (MS), reverse phase (RP) LC, size permeation (gel filtration), ion exchange, affinity, HPLC, UPLC and other LC or LC MS-based techniques. Appropriate LC MS techniques include ICAT^{®} (Applied Biosystems, CA, USA), or iTRAQ^{®} (Applied Biosystems, CA, USA). Liquid chromatography (*e.g*. high pressure liquid chromatography (HPLC) or low pressure liquid chromatography (LPLC)), thin-layer chromatography, NMR (nuclear magnetic resonance) spectroscopy could also be used.

Methods involving detection and/or quantification of one or more biomarkers can be performed on bench-top instruments, or can be incorporated onto disposable, diagnostic or monitoring platforms that can be used in a non-laboratory environment, *e.g*. in the physician's office or at the subject's bedside. Suitable biosensors for performing methods of the invention include "credit" cards with optical or acoustic readers. Biosensors can be configured to allow the data collected to be electronically transmitted to the physician for interpretation and thus can form the basis for e-medicine.

The identification of biomarkers for a disease state permits integration of diagnostic procedures and therapeutic regimes. The biomarkers provide the means to indicate therapeutic response, failure to respond, unfavourable side-effect profile, degree of medication compliance and achievement of adequate serum drug levels. The biomarkers may be used to provide warning of adverse drug response. Biomarkers are useful in development of personalized therapies, as assessment of response can be used to fine-tune dosage, minimise the number of prescribed medications, reduce the delay in attaining effective therapy and avoid adverse drug reactions. Thus by monitoring a biomarker, subject care can be tailored precisely to match the needs determined by the disorder and the pharmacogenomic profile of the subject, the biomarker can thus be used to titrate the optimal dose, predict a positive therapeutic response and identify those subjects at high risk of severe side effects. Biomarker-based tests provide a first line assessment of 'new' subjects, and provide objective measures for accurate and rapid diagnosis, not achievable using the current measures.

Biomarker monitoring methods, biosensors and kits are also vital as subject monitoring tools, to enable the physician to determine whether relapse is due to worsening of the disorder. If pharmacological treatment is assessed to be inadequate, then therapy can be reinstated or increased; a change in therapy can be given if appropriate. As the biomarkers are sensitive to the state of the disorder, they provide an indication of the impact of drug therapy.

References to "subject" or "patient" are used interchangeably herein. The subject may be a human or an animal subject. In one embodiment, the subject is a human. In one embodiment, the subject is a (non-human) animal. In one embodiment, the subject is a non-human mammal, such as a dog, mouse, rat or horse, in particular a dog. The use, panels and methods described herein may be performed *in vitro* or *ex vivo.*

In one embodiment, the subject is suspected of relapse to a vascular or haematological cancer. Minimal residual disease (MRD) is the name given to small numbers of leukaemic cells (cancer cells from the bone marrow) that remain in the person during treatment, or after treatment when the patient is in remission (*i.e*. patients with no symptoms or signs of disease). However, MRD is the major cause of relapse in cancer and leukaemia. Methods of the invention are therefore useful in monitoring patients who are suspected of relapse, particularly patients who are in remission from cancer.

The subject tested using the methods described herein may present with symptoms indicative of haematological cancer, for example anaemia, leucocytosis and/or swollen lymph nodes. In one embodiment, the subject has a high level of leucocytosis. This may also be referred to a "high white blood cell count". Haematological cancers typically cause increased proliferation of abnormal white or red blood cells which results in a high white blood cell count. However, leucocytosis is not sufficient to diagnose a patient with a haematological cancer (in particular leukaemia) because it is frequently a sign of an inflammatory response, most commonly the result of infection. Therefore, methods of the invention are able to provide a more specific method to detect patients who are likely to be suffering from a haematological cancer.

Detecting and/or quantifying may be compared to a cut-off level. Cut-off values can be predetermined by analysing results from multiple patients and controls, and determining a suitable value for classifying a subject as with or without the disease. For example, for diseases where the level of biomarker is higher in patients suffering from the disease, then if the level detected is higher than the cut-off, the patient is indicated to suffer from the disease. Alternatively, for diseases where the level of biomarker is lower in patients suffering from the disease, then if the level detected is lower than the cut-off, the patient is indicated to suffer from the disease. The advantages of using simple cut-off values include the ease with which clinicians are able to understand the test and the elimination of any need for software or other aids in the interpretation of the test results. Cut-off levels can be determined using methods in the art.

Detecting and/or quantifying may also be compared to a control. It will be clear to those skilled in the art that the control subjects may be selected on a variety of basis which may include, for example, subjects known to be free of the disease or may be subjects with a different disease (for example, for the investigation of differential diagnosis). The "control" may comprise a healthy subject, a non-diseased subject and/or a subject without a vascular or haematological cancer. Comparison with a control is well known in the field of diagnostics.

Therefore, in one embodiment, the method additionally comprises comparing the level of said cell free nucleosomes in said plasma sample with one or more controls. For example, the method may comprise comparing the level of cell free nucleosomes present in a plasma sample obtained from the subject with the level of cell free nucleosomes present in a plasma sample obtained from a normal subject. The control may be a healthy subject. Alternatively, the control may be a diseased subject, such as subject with an infection.

Alternatively, the control is a subject with a cancer which is not a vascular or haematological cancer, *i.e.* the control subject has a cancer which affects a different organ in the body. The data provided herein shows that biomarkers were significantly elevated in blood cancer patients compared to patients with other forms of cancer, therefore they may be used to differentially diagnose patients with vascular or haematological cancers over patients with other forms of cancer. Therefore, in one aspect the diagnosis comprises differential diagnosis of vascular or haematological cancer over non-vascular or non-haematological cancer. A "non-vascular or non-haematological cancer" is a cancer which is not a blood cancer and does not involve proliferation of the blood cells or vascular cells, such as bladder cancer, bone cancer, brain cancer, oesophageal cancer, head & neck cancer, skin cancer (such as melanoma), thyroid cancer, tongue cancer, uterine cancer and/or cervical cancer.

The control may be a subject with a high level of leucocytosis. As discussed herein, leucocytosis is not a symptom which is unique to leukaemia. Therefore, methods of the invention may be used to compare a control with a high level of leucocytosis which is not the result of a vascular or haematological cancer, such as a control with inflammation, an infection and/or taking medication.

In one embodiment, the level of cell free nucleosomes is elevated compared to the control.

It will be understood that it is not necessary to measure controls levels for comparative purposes on every occasion. For example, for healthy/non-diseased controls, once the 'normal range' is established it can be used as a benchmark for all subsequent tests. A normal range can be established by obtaining samples from multiple control subjects without a vascular or haematological cancer and testing for the level of biomarker. Results (*i.e*. biomarker levels) for subjects suspected to have a vascular or haematological cancer can then be examined to see if they fall within, or outside of, the respective normal range. Use of a 'normal range' is standard practice for the detection of disease.

In one embodiment, the method additionally comprises determining at least one clinical parameter for the patient. This parameter can be used in the interpretation of results. Clinical parameters may include any relevant clinical information for example, without limitation, gender, weight, Body Mass Index (BMI), smoking status and dietary habits. Therefore, in one embodiment, the clinical parameter is selected from the group consisting of: age, sex and body mass index (BMI).

In one embodiment, the method of the invention is performed to identify a subject at high risk of having a vascular or haematological cancer and therefore in need of further testing (*i.e*. further cancer investigations). The further testing may involve one or more of: biopsy (such as bone marrow biopsy or lymph node biopsy), cytogenetic testing, immunophenotyping, CT scanning, X-ray (in particular chest X-ray to identify swollen lymph nodes) and/or lumbar puncture.

Methods and biomarkers described herein may be used to identify if a patient is in need of a biopsy, in particular a bone marrow or lymph node biopsy. Therefore, also described is a method of identifying a patient in need of a biopsy comprising obtaining a plasma sample from said patient, detecting the level of cell free nucleosomes in the plasma sample, and using the results obtained from the panel test to identify whether the patient is in need of a biopsy.

### Additional biomarkers

The level of cell free nucleosomes may be detected or measured as one of a panel of measurements. The panel may comprise different epigenetic features of the nucleosome as described hereinbefore (*e.g*. a histone isoform and a PTM). In one embodiment, the panel comprises one or more cytokines, such as one or more interleukins.

Interleukins (ILs) are a group of cytokines, usually secreted by leukocytes, that act as signal molecules. They have key roles in stimulating immune responses and inflammation. They were first identified in the 1970s and have been designated numerically as more interleukin types have been discovered. Examples of interleukins include, but are not limited to: IL-1, IL-2, IL-3, IL-4, IL-5, IL-6, IL-7, IL-8, IL-9, IL-10, IL-11, IL-12, IL-13, IL-14 and IL-15.

In one embodiment, the one or more interleukins is selected from the group consisting of: Interleukin-6 (IL-6), Interleukin-10 (IL-10) and Interleukin-1β (IL-1β).

The interleukin may be IL-6. Interleukin-6 (IL-6) is a cytokine with a wide variety of biological functions. It is a potent inducer of fever and the acute phase response. The sequence of human IL-6 is known in the art and is described at UniProt Accession No. P05231. In one particular embodiment, the interleukin may be IL-6 and the panel of measurements may comprise measurement of histone isoform H3.1 and IL-6.

Alternatively, or additionally, the interleukin may be IL-10. Interleukin-10 (IL-10) is an antiinflammatory cytokine with a wide variety of biological functions. The sequence of human IL-10 is known in the art and is described at UniProt Accession No. P22301. In one particular embodiment, the interleukin may be IL-10 and the panel of measurements may comprise measurement of histone post-translational modification H3cit and IL-10.

Alternatively, or additionally, the interleukin may be IL-1β. Interleukin-1β (IL-1β) is a pro-inflammatory cytokine and is involved in a variety of cellular activities, including cell proliferation, differentiation, and apoptosis. In one particular embodiment, the interleukin may be IL-1β and the panel of measurements may comprise measurement of histone isoform H3.1 and IL-1β. More particularly, the panel may comprise measurement of histone isoform H3.1 and IL-1β, wherein the vascular or haematological cancer is lymphoma, such as NHL.

For example, the cell free nucleosome measurement can be combined with more than one interleukin measurement, such as IL-6 and IL-1β or IL-10 and IL-1β or IL-6 and IL-10. In a further embodiment, the panel of measurements is H3.1, IL-6 and IL-1β.

Models can be derived using the biomarkers. Methods for deriving models or algorithms such as those in Tables 5 and 6 of the Examples are well known in the art and suitable software packages are available. Typical software tools for this purpose include SPSS (Statistical Package for the Social Sciences) and "R". These software packages provide for linear and non-linear data modelling of clinical data.

It will be clear to those skilled in the art, that any combination of the biomarkers disclosed herein may be used in panels and algorithms for the detection of vascular or haematological cancer, and that further markers may be added to a panel including these markers.

It will be understood that the embodiments described herein may be applied to all aspects of the invention, *i.e.* the embodiment described for the uses may equally apply to the claimed methods and so forth.

The invention will now be illustrated with reference to the following non-limiting examples.

### EXAMPLES

### EXAMPLE 1

Plasma samples were taken from a cohort of 116 human subjects. For each subject, a whole blood draw was collected in an EDTA vacutainer tube and the tube was inverted 10 times gently. The whole blood was centrifuged for 15 minutes at 1500g within 2 hours of blood draw. Plasma was transferred to a cryotube and frozen immediately. In the cohort, 62 subjects were healthy, 25 subjects had Non-Hodgkin Lymphoma (NHL), 22 subjects had Acute Myeloid Leukaemia (AML) and 7 subjects had Acute Lymphoblastic Leukaemia (ALL). Subjects with leukaemia or lymphoma could be further categorised as patients with cancer at diagnosis (31 subjects in total; 16 subjects with NHL at diagnosis, 15 subjects with leukaemia at diagnosis), or patients with cancer from relapse (15 subjects in total; 6 subjects with NHL relapse, 9 subjects with leukaemia relapse).

The samples were analysed for nucleosomes containing H3.1 by ELISA. In brief, measurements of nucleosomes containing histone isoform H3.1 were made as follows: 80µl assay buffer and 20µl of plasma sample or standard nucleosome preparation was added to a microtiter well coated with an antibody directed to bind to histone H3.1. The microtiter plate was covered and incubated with gentle shaking for 2.5 hours at room temperature. The contents of the microtiter wells were discarded. The wells were washed three times with 200µl of a wash solution and 100µl of a biotinylated anti-nucleosome antibody was added. The microtiter plate was again covered and incubated with gentle shaking for 1.5 hours at room temperature. The contents of the microtiter wells were discarded. The wells were washed three times with 200µl of a wash solution and 100µl of a streptavidin-HRP solution was added. The microtiter plate was again covered and incubated with gentle shaking for 0.5 hours at room temperature. The contents of the microtiter wells were discarded. The wells were washed three times with 200µl of a wash solution and 100µl of a HRP (horse radish peroxidase) substrate solution was added. The microtiter plate was covered and incubated with gentle shaking for 20 minutes in the dark at room temperature. The absorbance (OD) of the wells was measured at 405nm. The OD levels were either used directly or the plasma level of nucleosomes containing histone H3.1 were interpolated from a standard curve.

The OD results were plotted as a Receiver Operator Characteristic (ROC) curve. Results were grouped for all patients with cancer vs. healthy, as well as all patients with lymphoma vs. healthy and all patients with leukaemia (i.e. including ALL and AML) vs. healthy. The area under the curve (AUC) results are shown in Tables 1 to 3.

**Table 1: Roc Curves for H3.1 OD in Plasma - All Patients**

| **Parameters** | **Cancer vs Healthy** | **NHL vs Healthy** | **Leukaemia vs Healthy** |
|---|---|---|---|
| Size | 54 Cancer vs 62 Healthy | 25 NHL vs 62 Healthy | 29 Leuk. vs 62 Healthy |
| AUC | 0.911 | 0.943 | 0.885 |
| Sens. for 80% Spec. | 81.5% | 88% | 75.9% |
| Sens. for 90% Spec. | 75.9% | 84% | 69% |
| Sens. for 95% Spec. | 74.1% | 80% | 69% |
| Sens. for 100% Spec. | 66.7% | 72% | 62.1% |
| R² | 66.6% | 72.9% | 62.2% |

**Table 2: Roc Curves for H3.1 OD in Plasma - Patients at Diagnosis**

| **Parameters** | **Cancer at diagnosis vs Healthy** | **NHL at diagnosis vs Healthy** | **Leukaemia at diagnosis vs Healthy** |
|---|---|---|---|
| Size | 31 Cancer vs 62 Healthy | 16 NHL vs 62 Healthy | 15 Leuk. vs 62 Healthy |
| AUC | 0.925 | 0.932 | 0.918 |
| Sens. for 80% Spec. | 83.9% | 87.5% | 80% |
| Sens. for 90% Spec. | 80.6% | 81.3% | 80% |
| Sens. for 95% Spec. | 80.6% | 81.3% | 80% |
| Sens. for 100% Spec. | 74.2% | 75% | 73.3% |
| R² | 71.1% | 70% | 70.1% |

**Table 3: Roc Curves for H3.1 OD in Plasma - Relapse Patients**

| **Parameters** | **Cancer relapse vs Healthy** | **NHL relapse vs Healthy** | **Leukaemia relapse vs Healthy** |
|---|---|---|---|
| Size | 15 Cancer vs 62 Healthy | 6 NHL vs 62 Healthy | 9 Leuk. vs 62 Healthy |
| AUC | 0.878 | 0.954 | 0.828 |
| Sens. for 80% Spec. | 80% | 83.3% | 77.8% |
| Sens. for 90% Spec. | 73.3% | 83.3% | 66.7% |
| Sens. for 95% Spec. | 73.3% | 83.3% | 66.7% |
| Sens. for 100% Spec. | 66.7% | 66.7% | 66.7% |
| R² | 62.2% | 69.4% | 54.1% |

The levels of cell free nucleosomes containing H3.1 were able to discriminate over 75% of blood cancer patients from healthy donors at 90% specificity, as shown in Table 1. This was able to increase to over 80% at 90% specificity for patients with cancer at diagnosis, as shown in Table 2. The results also indicated that the biomarker is particularly effective at identifying patients with lymphoma because 84% of patients with NHL were distinguished from healthy subjects at 90% specificity (see Table 1).

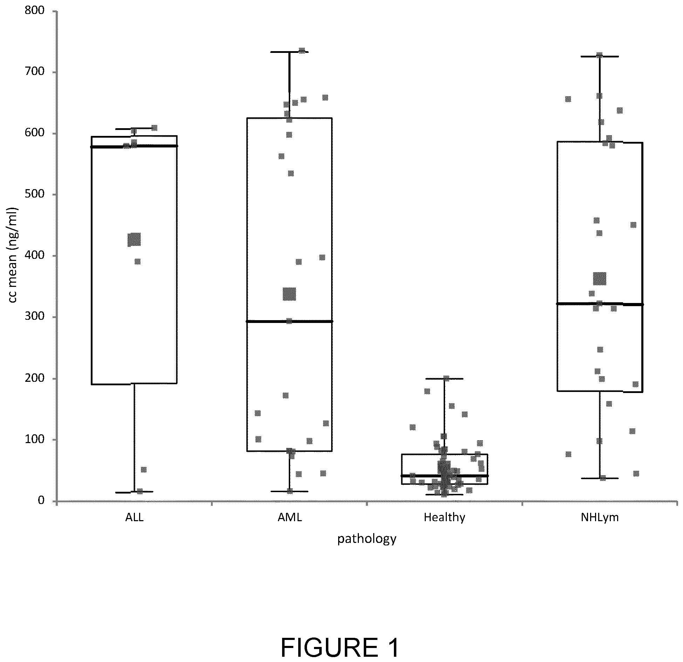

Concentrations of H3.1, derived from the OD values using a standard curve for interpolation, in plasma samples for all AML, ALL and NHL patients compared to the level in healthy patients is shown in **Figure 1**. There is clear elevation in levels of H3.1 in patients with haematological cancers compared to healthy subjects.

The results show that nucleosomes levels, particularly nucleosomes containing H3.1, can be used for haematological cancer detection.

### EXAMPLE 2

The level of cell free nucleosomes containing H3.1 in human plasma samples tested in Example 1 were further compared to H3.1 levels in plasma samples obtained from human patients with other forms of cancer, also collected as described in Example 1.

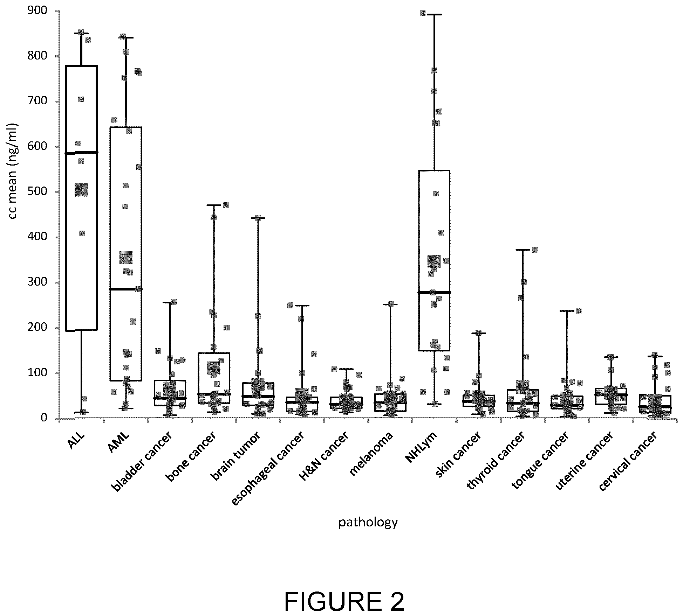

The results are shown in **Figure 2**. Surprisingly, it was found the H3.1 level could be used to differentiate between subjects with haematological cancer vs. patients with other forms of cancer. H3.1 levels were significantly elevated in patients for each type of blood cancer (ALL, AML and NHL) compared to H3.1 levels in plasma samples from patients with bladder, bone, brain, oesophageal, head & neck, skin, thyroid, tongue, uterine and cervical cancer and melanoma.

### EXAMPLE 3

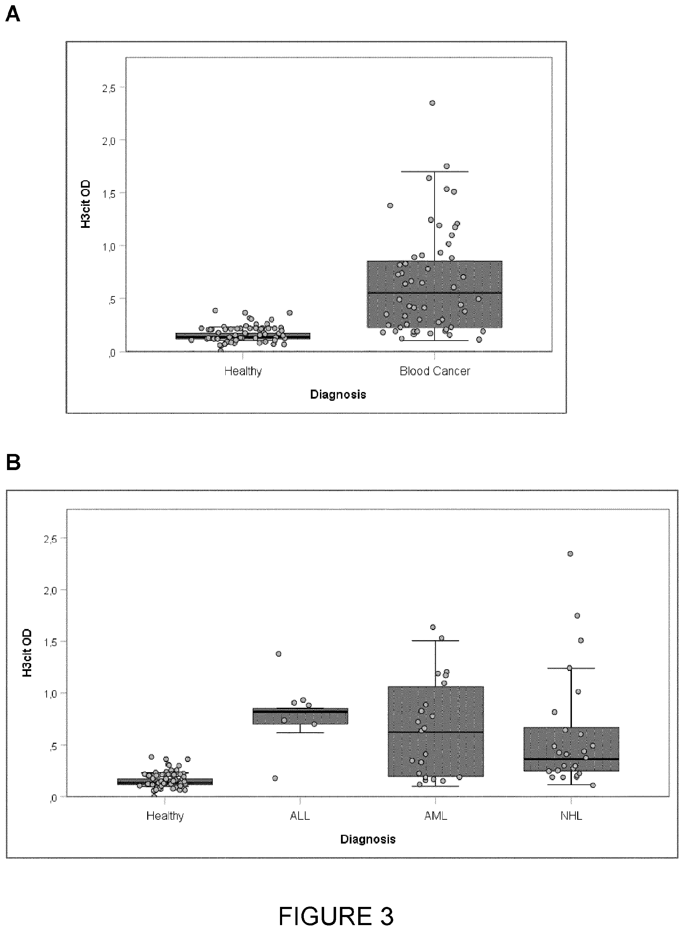

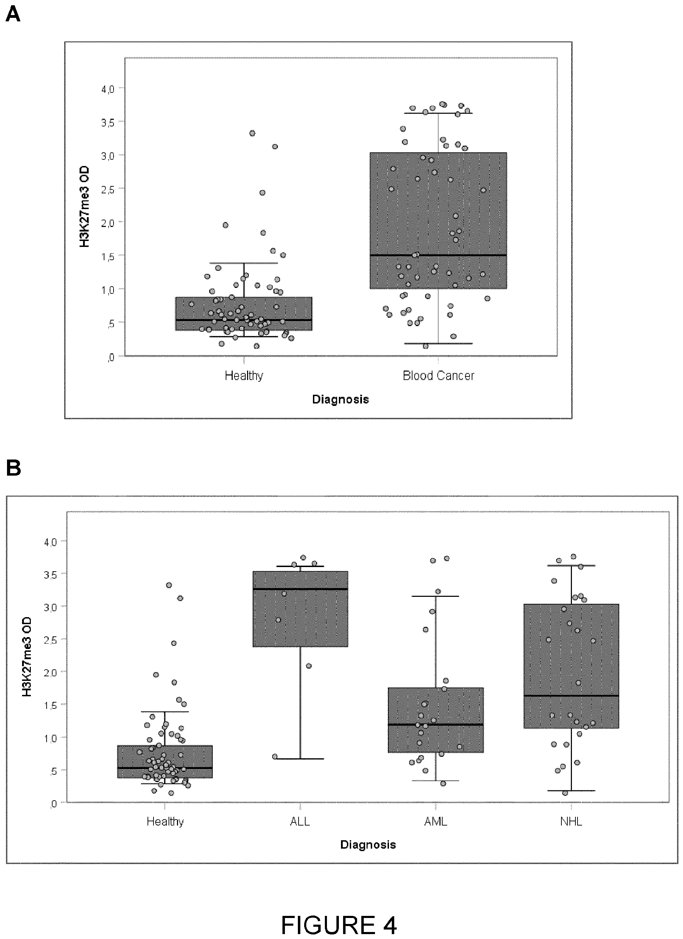

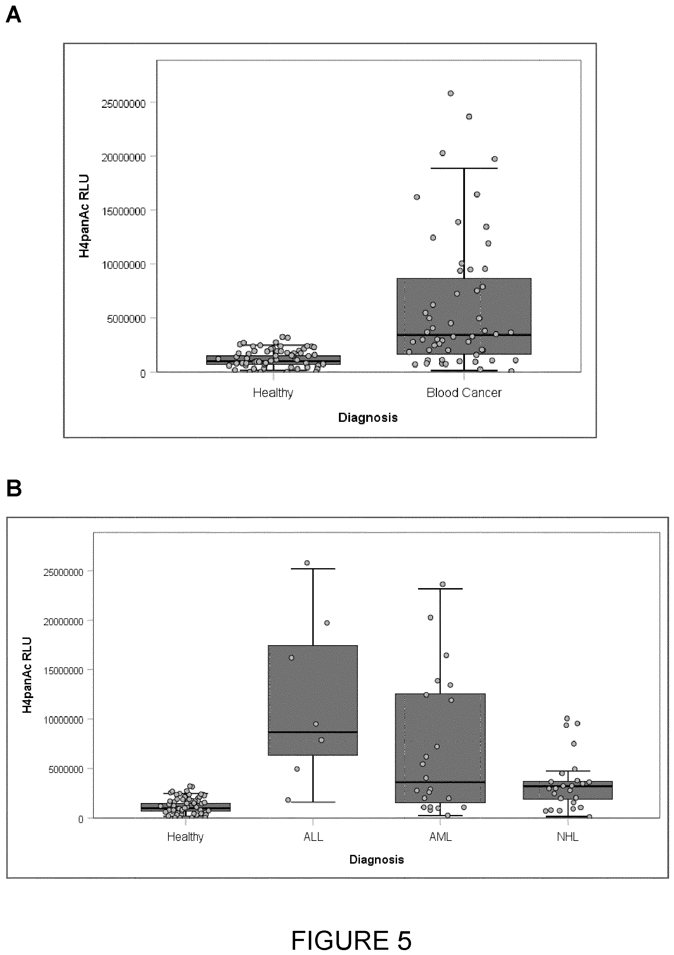

Levels of cell free nucleosomes with post translational modifications was tested on the human plasma samples described in Example 1. ELISA methods were run in a similar manner to the ELISA method for H3.1 as described in Example 1, except the antibody directed to bind to histone H3.1 was replaced with an antibody directed to a post translational histone modification selected from H3cit, H3K27Me3 and H4panAc. The results are summarised in Table 4. The results are also presented graphically for all blood cancers and for each blood cancer type (see **Figures 3-5**).

**Table 4: Roc Curves for PTM Nucleosomes in Plasma - all Blood Cancers**

| **Biomarkers** | **AUC** | **Sensitivity 80% Specif.** | **Sensitivity 90% Specif.** | **Size** | **R²** |
|---|---|---|---|---|---|
| H3cit OD | 0.913 | 85.2% | 77.8% | 54 Cancer vs 62 Healthy | 66% |
| H3K27Me3 OD | 0.822 | 72.2% | 50% | 54 Cancer vs 62 Healthy | 36.8% |
| H4panAc RLU | 0.847 | 77.8% | 68.5% | 54 Cancer vs 62 Healthy | 55.8% |

All nucleosome post translational modifications tested show a significant elevation in patients with blood cancers compared to healthy controls. Even when blood cancers are separated by type (ALL, AML, NHL), there is a significant elevation of the levels of H3cit, H3K27Me3 and H4panAc cell free nucleosomes in plasma samples compared to healthy controls (see **Figures 3B**, **4B** and **5B**)**.** Levels of post translationally modified nucleosomes were elevated the most in plasma samples obtained from patients with ALL.

### EXAMPLE 4

The two best cell free nucleosome markers (H3.1 and H3cit) were combined together or in combination with measurements of different interleukins. Cell free nucleosome levels were measured as described before and plasma interleukin levels were measured using commercially available ELISA methods.

Assay results were modelled by Logistic Regression analysis to train for the model or algorithm with the highest AUC for a comparison of human patients with blood cancer vs normal human donors. The results are summarised in Table 5.

**Table 5: Roc Curves for Combined Biomarker Panels in Plasma - all Blood Cancers^{‡}**

| **Biomarker Models** | **AUC** | **Sensitivity 80% Specif.** | **Sensitivity 90% Specif.** | **R²** |
|---|---|---|---|---|
| Model 01: LnY = H3cit | 0.913 | 85.2% | 77.8% | 66% |
| Model 02: LnY = H3.1 | 0.911 | 81.5% | 75.9% | 66.6% |
| Model 03: LnY = H3.1 + H3cit | 0.921 | 88.9% | 81.5% | 68.1% |
| Model 04: LnY = H3cit + H3K27me3 | 0.923 | 85.2% | 81.5% | 68.3% |
| Model 05: LnY = H3.1 + hIL6 | 0.935 | 87% | 75.9% | 69.4% |
| Model 06: LnY = H3cit + hIL10 | 0.930 | 87% | 79.6% | 69.6% |
| Model 07: LnY = H3cit + hIL1β + hIL10 | 0.935 | 90.7% | 85.2% | 73.2% |
| Model 08: LnY = H3.1 + hIL1β + hIL6 | 0.936 | 92.6% | 75.9% | 71% |

| | | | | |
|---|---|---|---|---|
| * Cohort size: 54 Cancer vs 62 Healthy | | | | |

All models were able to discriminate over 75% of blood cancer patients from healthy donors at 90% specificity, as shown in Table 5. The results show that nucleosomes levels can be combined with interleukin levels as an effective assay panel with an associated algorithm for haematological cancer detection.

### EXAMPLE 5

Plasma samples from only those human patients with NHL were modelled by Logistic Regression analysis as described in Example 4. Results are summarised in Table 6.

**Table 6: Roc Curves for Combined Biomarker Panels in Plasma - NHL Cancers^{‡}**

| **Biomarker Models** | **AUC** | **Sensitivity 80% Specif.** | **Sensitivity 90% Specif.** | **R²** |
|---|---|---|---|---|
| Model 01: LnY = H3cit | 0.919 | 84% | 80% | 65.3% |
| Model 02: LnY = H3.1 | 0.943 | 88% | 84% | 72.9% |
| Model 03: LnY = H3.1 + hIL1β | 0.979 | 100% | 92% | 83.6% |

| | | | | |
|---|---|---|---|---|
| ^{‡} Cohort size: 25 NHL vs 62 Healthy | | | | |

All models were able to discriminate at 80% or more patients with NHL from healthy donors at 90% specificity, as shown in Table 6. In particular, combining H3.1 and IL-1β was able to discriminate 100% of patients with NHL at 80% specificity. The results show that nucleosome and interleukin levels can be used as an effective assay panel with an associated algorithm for NHL detection.

### EXAMPLE 6

Plasma samples were taken from 73 dogs diagnosed with canine hemangiosarcoma, 127 dogs diagnosed with canine lymphoma and 134 control dogs with no cancer. The samples were analysed for nucleosomes containing H3.1 by ELISA.

Healthy dogs were found to have a uniformly low concentration of circulating nucleosomes containing histone isoform H3.1 of less than 67.4 ng/ml (mean 32 ng/ml, median 31 ng/ml).

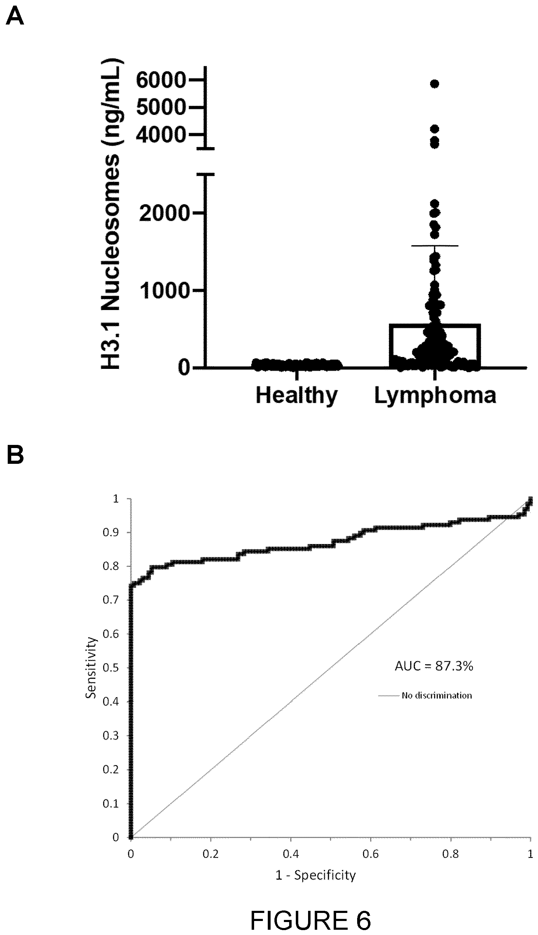

Dogs diagnosed with lymphoma were found to have highly elevated levels of circulating nucleosomes containing histone isoform H3.1 (mean 570 ng/ml, median 211 ng/ml). A dot-plot and a ROC curve of the results for obtained for canine lymphoma are shown graphically in Figure 6A and 6B respectively. The AUC for detection of lymphoma was 87%. Using a cut-off of 67.4 ng/ml, the specificity of the assay was 100% with a sensitivity of 74%. Using a lower cut-off of 48.1 ng/ml gave a sensitivity of 81% at a specificity of 90%.

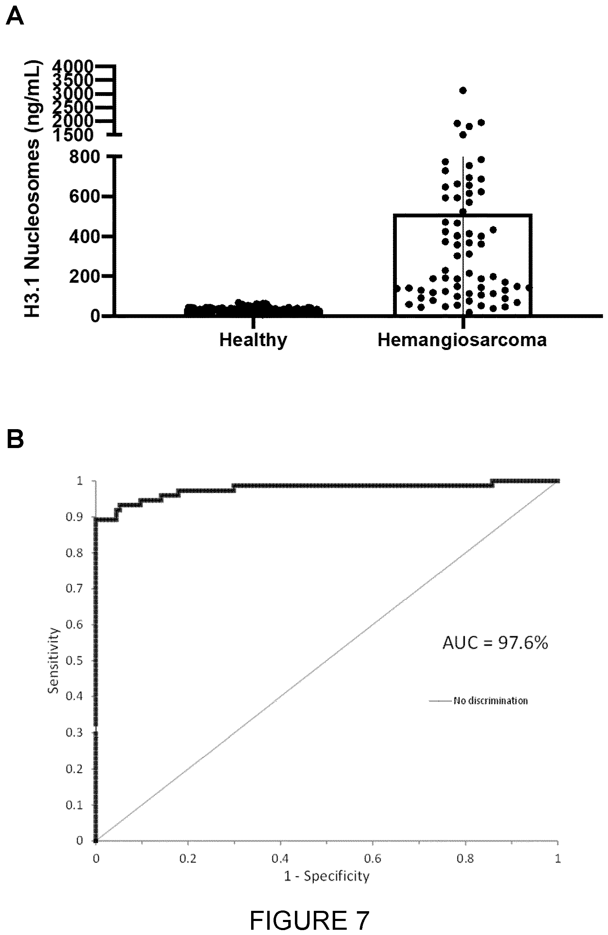

Dogs diagnosed with hemangiosarcoma were also found to have highly elevated levels of circulating nucleosomes containing histone isoform H3.1 (mean 513 ng/ml, median 361 ng/ml). A dot-plot and a ROC curve of the results obtained for canine hemangiosarcoma are shown graphically in Figure 7A and 7B respectively. The AUC for detection of hemangiosarcoma was 97.6%. Using a cut-off of 67.4 ng/ml, the specificity of the assay was 100% with a sensitivity of 89%. Using a lower cut-off of 48.1 ng/ml gave a sensitivity of 95% at a specificity of 90%.

As we found in human disease, the level of circulating nucleosomes observed for dogs diagnosed with lymphoma or hemangiosarcoma was also much higher than was observed for other canine tumours investigated.

Most assays for human proteins are not transferable to other animals. However, the structure of nucleosomes is highly conserved across species and even phyla. We have shown that this means that this assay for human H3.1 nucleosomes is transferable to other species including dogs and horses. Moreover, the results we observed for vascular and hematopoietic cancers in canine subjects are remarkably similar to the results observed in human subjects. We conclude that the methods of the invention are highly effective methods for the detection of both human and animal vascular and haematopoietic cancers.

### EXAMPLE 7

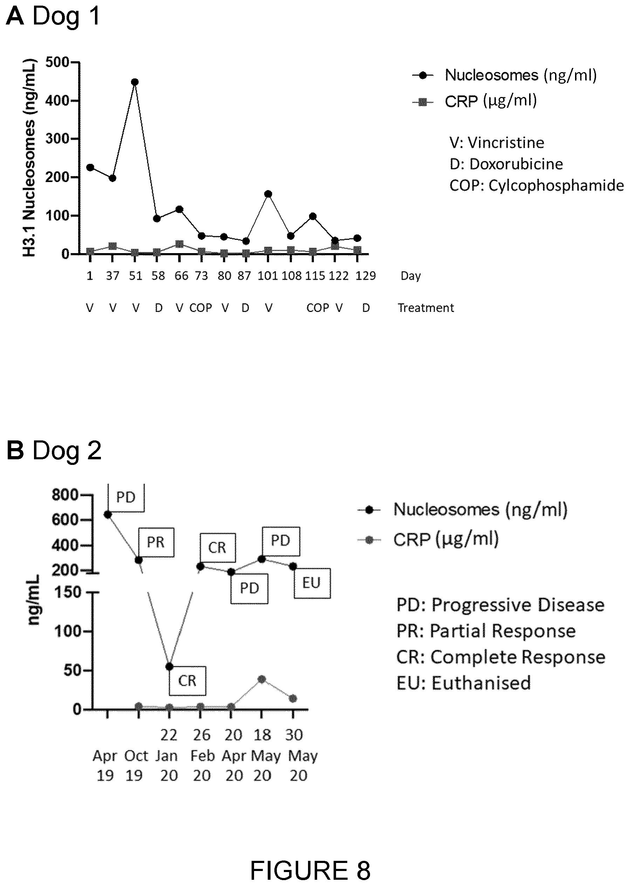

Serial plasma samples were taken from 2 dogs undergoing treatment for hemangiosarcoma and 2 dogs undergoing treatment for lymphoma. All the samples from the 4 dogs were collected and later analysed for nucleosomes containing histone isoform H3.1 after the last day of treatment in Figure 8 so that the information did not inform clinical treatment decisions, which were made on clinical grounds.

C-Reactive Protein (CRP) is a well-known biomarker for inflammation and this was also measured in the samples as a control to determine whether nucleosome levels simply reflected an inflammatory response or provided additional information regarding subject status, prognosis and treatment response.

Dog 1 (Figure 8A) was diagnosed with hemangiosarcoma which was treated by a chemotherapy regimen of 4 drugs known as CHOP (cyclophosphamide, doxorubicin, vincristine, and prednisone) commencing day 1 through to day 129. The treatment was successful and dog 1 was determined to be in remission on clinical grounds on day 160. The success of the CHOP treatment is reflected in the downward trend in nucleosome levels over the treatment course and disease remission was predicted by the near normal nucleosome levels by day 122. The results demonstrate that nucleosome levels are useful as a prognostic indicator and can be used to guide treatment regimens and to monitor subjects in remission for disease relapse. CRP analysis was not useful for clinical purposes.

Dog 2 (Figure 8B) was initially diagnosed with hemangiosarcoma in 2017 and was treated with doxorubicin and an immunomodulatory agent. He was monitored regularly by whole body CT scan every 2-3 months. He was noted to have recurrence 2 years later and this is reflected in a high level of circulating nucleosomes. The recurrence was treated successfully with doxorubicin, dacarbazine and an immunomodulatory agent as well as whole lung radiation and stereotactic body radiotherapy (SBRT) and dog 2 was determined to be in remission based on whole body CT imaging. The success of the various treatments is reflected in the near normal nucleosome levels measured in January 2020. In February of 2020 dog 2 was diagnosed on imaging grounds as having a complete response, but interestingly the measured nucleosome levels began to rise in February before the imaging was able to detect progressive disease at the next scan in April 2020. The nucleosome levels remained high when progressive disease was noted in April. Had the nucleosome levels been known at the time of treatment, more intense monitoring would have been established for this patient in February. The results demonstrate that nucleosome levels can be used to guide treatment regimens and to monitor subjects in remission for disease relapse. CRP analysis was not useful for clinical purposes.

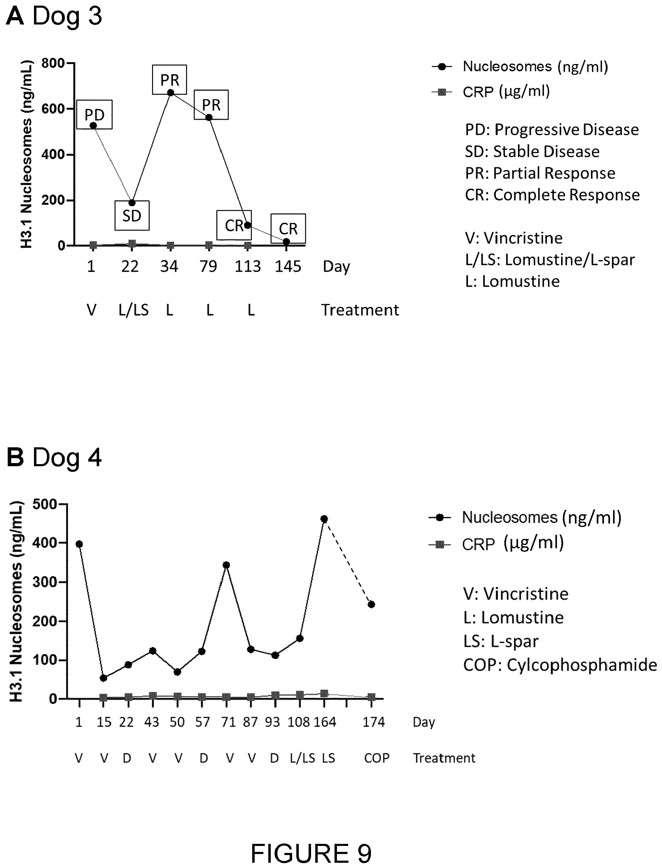

Dog 3 (Figure 9A) was initially diagnosed and successfully treated for lymphoma in 2018 but came out of remission and this was reflected in the measured nucleosome levels. He was treated on day 1 with vincristine for Progressive Disease (PD) as part of a CHOP chemotherapy regimen. The vincristine treatment led to an improvement in clinical condition to Stable Disease (SD) which was reflected in a fall in nucleosome level as shown by the results in Figure 9A. However, CHOP treatment was discontinued due to both toxicity reasons and a lack of robust observed clinical response. Treatment with lomustine plus L-asparaginase (L-spar) was initiated on day 22 and followed by further lomustine treatments on days 34, 79, and 113. The clinical findings improved to partial remission on day 34 and 79 as determined through tumour measurements and then to complete response (CR) to treatment on days 113 and 145 by which time dog 3 was in remission and no further chemotherapy was administered. Nucleosome levels rose between days 22-34 and then fell consistently from day 34 onwards, predicting the success of the lomustine treatment later observed clinically as complete response on days 113 and 145. Moreover, nucleosome levels fell into the range observed for healthy dogs on day 145 in accord with the clinical findings. CRP levels were always within the normal range and were not useful for clinical purposes.

Dog 4 (Figure 9B) was diagnosed with Stage Vb intra-abdominal, hypercalcaemic, lymphoma. He was started on a CHOP chemotherapy regime of vincristine and doxorubicin on day 1 through to day 93 and his response to treatment was monitored based on circulating calcium levels. It was appreciated clinically by the veterinarians that there was some response to vincristine but no response to doxorubicin. Treatment was then switched from CHOP to lomustine plus L-spar on days 108-164 which he also did not respond to. As a response to vincristine had been noticed, treatment by COP (CHOP without the use of doxorubicin) was initiated on day 164 using vincristine (a blood sample could not be taken on day 167). Dog 4 responded to COP therapy and was still responding to treatment at the time of writing. The measured nucleosome levels reflect the observed clinical findings (Figure 9B). There was a decrease in nucleosome level at most time points following treatment with vincristine reflecting the response to this therapy and an increase in nucleosome level after each dose of doxorubicin reflecting the lack of response to this treatment. Dog 4 was also clinically found to have a partial response to cyclophosphamide which was also reflected in a decreased nucleosome concentration. However, nucleosome levels remained above normal levels indicating the continuing need for further treatment. Nucleosome measurements clearly correlate to clinical findings and can be used to monitor disease remission and to guide treatment selection. For example, nucleosome levels predicted the lack of response to doxorubicin therapy which may have been discontinued earlier if informed by nucleosome levels. CRP analysis did not show any significant variation and was not useful for clinical purposes.

## Claims

1. Use of a H3.1 histone isoform of a cell free nucleosome as a biomarker in a plasma sample, for the diagnosis or detection of a vascular or haematological cancer.

2. The use as defined in claim 1, wherein the cell free nucleosome is a mononucleosome or oligonucleosome.

3. The use as defined in claim 1 or claim 2, wherein the haematological cancer is selected from leukaemia, lymphoma or myeloma.

4. The use as defined in claim 3, wherein the leukaemia is selected from Acute Lymphocytic Leukaemia (ALL) or Acute Myeloid Leukaemia (AML), or wherein the lymphoma is Non-Hodgkin Lymphoma (NHL).

5. The use as defined in claim 1 or claim 2, wherein the vascular cancer is selected from angiosarcoma or hemangiosarcoma.

6. A method for diagnosing or detecting a vascular or haematological cancer which comprises the steps of:
(i) contacting a plasma sample obtained from the subject with a binding agent to detect or measure cell free nucleosomes containing histone isoform H3.1; and
(ii) using the level of cell free nucleosomes containing histone isoform H3.1 detected to diagnose the subject with the vascular or haematological cancer.

7. A method for determining the prognosis of a subject with a vascular or haematological cancer, which comprises the steps of:
(i) contacting a plasma sample obtained from the subject with a binding agent to detect or measure cell free nucleosomes containing histone isoform H3.1; and
(ii) using the level of cell free nucleosomes containing histone isoform H3.1 detected as indicative of the prognosis of said vascular or haematological cancer.

8. A method for monitoring the efficacy of a therapy in a subject having, suspected of having, or being predisposed to a vascular or haematological cancer, which comprises the steps of:
(i) contacting a plasma sample obtained from the subject with a binding agent to detect or measure cell free nucleosomes containing histone isoform H3.1; and
(ii) comparing the level of cell free nucleosomes containing histone isoform H3.1 detected with an earlier plasma sample taken from said subject to determine the efficacy of said therapy.

9. The method as defined in any one of claims 6 to 8, wherein said detection or measurement comprises an immunoassay, immunochemical, mass spectroscopy, chromatographic, chromatin immunoprecipitation or biosensor method.

10. The method as defined in any one of claims 6 to 9, wherein said detection or measurement comprises a 2-site immunoassay employing a labelled anti-nucleosome detection binding agent in combination with an immobilized anti-histone H3.1 binding agent.

11. The method as defined in any one of claims 6 to 10, wherein the subject is a human or an animal subject.

12. The method as defined in any one of claims 6 to 11, wherein the subject is suspected of relapse to a vascular or haematological cancer, and/or wherein the subject has a high level of leucocytosis/a high white blood cell count.

13. The method as defined in any one of claims 6 to 12, additionally comprising comparing the level of said cell free nucleosomes containing histone isoform H3.1 in said plasma sample with one or more controls.

14. The method as defined in claim 13, wherein the control is a healthy subject, a subject with a cancer which is not a vascular or haematological cancer, or a subject with a high level of leucocytosis.

15. The method as defined in any one of claims 6 to 14, wherein the level of cell free nucleosomes containing histone isoform H3.1 is elevated compared to the control.

16. The method as defined in any one of claims 6 to 15, wherein the level of cell free nucleosomes containing histone isoform H3.1 is detected or measured as one of a panel of measurements, for example wherein the panel comprises one or more interleukins such as IL-6, IL-10 and IL-1β.

## Patentansprüche

1. Verwendung einer H3.1-Histonisoform eines zellfreien Nukleosoms als Biomarker in einer Plasmaprobe zur Diagnose oder zum Nachweis eines vaskulären oder hämatologischen Krebses.

2. Verwendung nach Anspruch 1, wobei das zellfreie Nukleosom ein Mononukleosom oder ein Oligonukleosom ist.

3. Verwendung nach Anspruch 1 oder Anspruch 2, wobei der hämatologische Krebs ausgewählt ist aus Leukämie, Lymphom oder Myelom.

4. Verwendung nach Anspruch 3, wobei die Leukämie ausgewählt ist aus akuter lymphatischer Leukämie (ALL) oder akuter myeloischer Leukämie (AML), oder wobei das Lymphom Non-Hodgkin-Lymphom (NHL) ist.

5. Verwendung nach Anspruch 1 oder Anspruch 2, wobei der Gefäßkrebs ausgewählt ist aus Angiosarkom oder Hämangiosarkom.

6. Verfahren zum Diagnostizieren oder zum Nachweisen eines vaskulären oder hämatologischen Krebses, das die folgenden Schritte umfasst:
(i) Inkontaktbringen einer von dem Probanden erhaltenen Plasmaprobe mit einem Bindemittel zum Nachweis oder zur Messung von zellfreien Nukleosomen, die Histonisoform H3.1 enthalten; und
(ii) Verwendung des Gehalts an zellfreien Nukleosomen, die nachgewiesenes Histonisoform H3.1 enthalten, zur Diagnose des vaskulären oder hämatologischen Krebses.

7. Verfahren zum Bestimmen der Prognose eines Probanden mit einem vaskulären oder hämatologischen Krebs, welches die Schritte umfasst:
(i) Inkontaktbringen einer von dem Probanden erhaltenen Plasmaprobe mit einem Bindemittel zum Nachweis oder zur Messung von zellfreien Nukleosomen, die Histonisoform H3.1 enthalten; und
(ii) unter Verwenden des Spiegels von zellfreien Nukleosomen, die nachgewiesenes Histonisoform H3.1 enthalten, als Indikator für die Prognose des genannten vaskulären oder hämatologischen Krebses.

8. Verfahren zum Überwachen der Wirksamkeit einer Therapie bei einem Probanden, der einen vaskulären oder hämatologischen Krebs aufweist, vermutet oder dafür prädisponiert ist, umfassend die Schritte:
(i) Inkontaktbringen einer von dem Probanden erhaltenen Plasmaprobe mit einem Bindemittel zum Nachweis oder zur Messung von zellfreien Nukleosomen, die Histonisoform H3.1 enthalten; und
(ii) Vergleichen des Gehalts an zellfreien Nukleosomen, die nachgewiesenes Histonisoform H3.1 enthalten, mit einer früheren Plasmaprobe, die von dem Probanden entnommen wurde, um die Wirksamkeit der Therapie zu bestimmen.

9. Verfahren nach einem der Ansprüche 6 bis 8, wobei der Nachweis oder die Messung ein Immunoassay-, immunchemisches, Massenspektroskopie-, chromatographisches, Chromatin-Immunpräzipitation- oder Biosensorverfahren umfasst.

10. Verfahren nach einem der Ansprüche 6 bis 9, wobei der Nachweis oder die Messung einen 2-Site-Immunoassay umfasst, der ein markiertes Anti-Nukleosomen-Detektions-Bindemittel in Kombination mit einem immobilisierten Anti-Histon H3.1-Bindemittel verwendet.

11. Verfahren nach einem der Ansprüche 6 bis 10, wobei der Proband ein Mensch oder ein Tier ist.

12. Verfahren nach einem der Ansprüche 6 bis 11, wobei der Proband im Verdacht steht, in einen vaskulären oder hämatologischen Krebs zurückzufallen, und/oder wobei der Proband einen hohen Grad an Leukozytose/eine hohe Anzahl weißer Blutkörperchen aufweist.

13. Verfahren nach einem der Ansprüche 6 bis 12, das zusätzlich das Vergleichen des Spiegels der zellfreien Nukleosomen, die Histonisoform H3.1 in der Plasmaprobe enthalten, mit einer oder mehreren Kontrollen umfasst.

14. Verfahren nach Anspruch 13, wobei die Kontrolle ein gesunder Proband, ein Proband mit einem Krebs, der kein vaskulärer oder hämatologischer Krebs ist, oder ein Proband mit einem hohen Gehalt an Leukozytose ist.

15. Verfahren nach einem der Ansprüche 6 bis 14, wobei der Gehalt an zellfreien Nukleosomen, die die Histonisoform H3.1 enthalten, im Vergleich zur Kontrolle erhöht ist.

16. Verfahren nach einem der Ansprüche 6 bis 15, wobei der Spiegel von zellfreien Nukleosomen, die Histonisoform H3.1 enthalten, als eines von einem Panel von Messungen detektiert oder gemessen wird, beispielsweise wobei das Panel ein oder mehrere Interleukine wie IL-6, IL-10 und IL-1β umfasst.

## Revendications

1. Utilisation d'une isoforme d'histone H3.1 d'un nucléosome acellulaire comme biomarqueur dans un échantillon de plasma, pour le diagnostic ou la détection d'un cancer vasculaire ou hématologique.

2. Utilisation selon la revendication 1, dans laquelle le nucléosome acellulaire est un mononucléosome ou un oligonucléosome.

3. Utilisation selon la revendication 1 ou la revendication 2, dans laquelle le cancer hématologique est choisi parmi la leucémie, le lymphome ou le myélome.

4. Utilisation selon la revendication 3, dans laquelle la leucémie est choisie parmi la leucémie lymphoïde aiguë (LLA) ou la leucémie myéloïde aiguë (LMA), ou dans laquelle le lymphome est un lymphome non hodgkinien (LNH).

5. Utilisation selon la revendication 1 ou la revendication 2, dans laquelle le cancer vasculaire est choisi parmi l'angiosarcome ou l'hémangiosarcome.

6. Procédé de diagnostic ou de détection d'un cancer vasculaire ou hématologique qui comprend les étapes de :
(i) mise en contact d'un échantillon de plasma obtenu auprès du sujet avec un agent de liaison pour détecter ou mesurer des nucléosomes acellulaires contenant une isoforme d'histone H3.1 ; et
(ii) utilisation du niveau de nucléosomes acellulaires contenant une isoforme d'histone H3.1 détecté pour diagnostiquer le sujet atteint du cancer vasculaire ou hématologique.

7. Procédé de détermination du pronostic d'un sujet atteint d'un cancer vasculaire ou hématologique, qui comprend les étapes de :
(i) mise en contact d'un échantillon de plasma obtenu auprès du sujet avec un agent de liaison pour détecter ou mesurer des nucléosomes acellulaires contenant une isoforme d'histone H3.1 ; et
(ii) utilisation du niveau de nucléosomes acellulaires contenant une isoforme d'histone H3.1 détecté comme indicatif du pronostic dudit cancer vasculaire ou hématologique.

8. Procédé de surveillance de l'efficacité d'une thérapie chez un sujet atteint, suspecté d'avoir ou étant prédisposé à un cancer vasculaire ou hématologique, qui comprend les étapes de :
(i) mise en contact d'un échantillon de plasma obtenu auprès du sujet avec un agent de liaison pour détecter ou mesurer des nucléosomes acellulaires contenant une isoforme d'histone H3.1 ; et
(ii) comparaison du niveau de nucléosomes acellulaires contenant une isoforme d'histone H3.1 détecté avec un échantillon de plasma antérieur prélevé auprès dudit sujet pour déterminer l'efficacité de ladite thérapie.

9. Procédé selon l'une quelconque des revendications 6 à 8, dans lequel ladite détection ou mesure comprend un procédé de dosage immunologique, immunochimique, de spectroscopie de masse, chromatographique, d'immunoprécipitation de la chromatine ou de biocapteur.

10. Procédé selon l'une quelconque des revendications 6 à 9, dans lequel ladite détection ou mesure comprend un dosage immunologique à 2 sites employant un agent de liaison de détection anti-nucléosome marqué en combinaison avec un agent de liaison anti-histone H3.1 immobilisé.

11. Procédé selon l'une quelconque des revendications 6 à 10, dans lequel le sujet est un sujet humain ou animal.

12. Procédé selon l'une quelconque des revendications 6 à 11, dans lequel le sujet est suspecté d'une rechute d'un cancer vasculaire ou hématologique, et/ou dans lequel le sujet présente un niveau élevé de leucocytose/un nombre élevé de globules blancs.

13. Procédé selon l'une quelconque des revendications 6 à 12, comprenant également la comparaison du niveau desdits nucléosomes acellulaires contenant une isoforme d'histone H3.1 dans ledit échantillon de plasma avec un ou plusieurs témoins.

14. Procédé selon la revendication 13, dans lequel le témoin est un sujet sain, un sujet atteint d'un cancer qui n'est pas un cancer vasculaire ou hématologique, ou un sujet présentant un niveau élevé de leucocytose.

15. Procédé selon l'une quelconque des revendications 6 à 14, dans lequel le niveau de nucléosomes acellulaires contenant une isoforme d'histone H3.1 est élevé par rapport au témoin.

16. Procédé selon l'une quelconque des revendications 6 à 15, dans lequel le niveau de nucléosomes acellulaires contenant une isoforme d'histone H3.1 est détecté ou mesuré comme l'un d'un panel de mesures, par exemple dans lequel le panel comprend une ou plusieurs interleukines telles que IL-6, IL-10 et IL-1β.
